(19) **Europäisches Patentamt / European Patent Office / Office européen des brevets**

(11) **EP 3 200 760 B1**

(12) **FASCICULE DE BREVET EUROPEEN**

(45) Date de publication et mention de la délivrance du brevet:
**19.08.2020 Bulletin 2020/34**

(21) Numéro de dépôt: **15784077.8**

(22) Date de dépôt: **30.09.2015**

(51) Int Cl.:
*A61K 8/81* (2006.01)    *A61Q 19/00* (2006.01)
*A61K 8/06* (2006.01)

(86) Numéro de dépôt international:
**PCT/FR2015/052615**

(87) Numéro de publication internationale:
**WO 2016/051086 (07.04.2016 Gazette 2016/14)**

(54) **COMPOSITION COSMÉTIQUE ET/OU PHARMACEUTIQUE SOUS FORME DE DISPERSION, PROCÉDÉ DE PRÉPARATION ET UTILISATION POUR LE TRAITEMENT DE LA PEAU**

KOSMETISCHE UND/ODER PHARMAZEUTISCHE ZUSAMMENSETZUNG IN FORM EINER DISPERSION, VERFAHREN ZUR HERSTELLUNG DAVON UND VERWENDUNG DAVON ZUR HAUTBEHANDLUNG

COSMETIC AND/OR PHARMACEUTICAL COMPOSITION IN THE FORM OF A DISPERSION, METHOD FOR PREPARING SAME AND USE THEREOF FOR SKIN TREATMENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.10.2014 FR 1459434**

(43) Date de publication de la demande:
**09.08.2017 Bulletin 2017/32**

(73) Titulaire: **Pierre Fabre Dermo-Cosmétique**
**92100 Boulogne-Billancourt (FR)**

(72) Inventeurs:
• **BIDAN, Catherine**
  **F-31 860 Labarthe sur Leze (FR)**
• **CELLIER, Dominique**
  **F-31270 Frouzins (FR)**
• **RATTIER, Sandy**
  **F-31130 Flourens (FR)**
• **MOULIS, Laure**
  **F-31450 Montlaur (FR)**

(74) Mandataire: **Ipside**
**6, Impasse Michel Labrousse**
**31100 Toulouse (FR)**

(56) Documents cités:
**EP-A1- 1 920 756      FR-A1- 2 902 660**
**FR-A1- 2 963 250      FR-A1- 2 977 801**
**US-A1- 2013 108 572**

**Description**

La présente invention s'inscrit dans le domaine du soin, de la protection et/ou du traitement de la peau, des muqueuses et/ou des cheveux. Plus particulièrement, elle concerne une composition cosmétique, dermatologique et/ou pharmaceutique, en particulier destinée à un usage topique, sous forme d'une dispersion d'une phase interne discontinue dans une phase externe continue, utilisable pour un tel soin et/ou traitement, ainsi qu'un procédé de préparation d'une telle composition.

Pour des raisons liées notamment à leur confort d'utilisation, les compositions cosmétiques, dermatologiques et/ou pharmaceutiques destinées à une application par voie topique se présentent très fréquemment sous forme d'émulsions. Ces émulsions peuvent être du type eau-dans-huile, mais sont le plus souvent de type huile-dans-eau, c'est-à-dire comportant une phase huileuse maintenue dispersée de manière homogène dans une phase aqueuse à l'aide d'un agent tensioactif.

Les compositions cosmétiques, dermatologiques et/ou pharmaceutiques sous forme d'émulsions à usage topique classiquement proposées pour le soin, la protection et/ou le traitement de la peau comportent de nombreux ingrédients, en particulier des agents tensioactifs destinés à stabiliser les émulsions, ainsi que des conservateurs visant à les préserver de toute prolifération microbienne pendant des périodes relativement longues, en conditionnement multidoses. Cependant, de tels ingrédients s'avèrent irritants pour la peau, et en particulier pour les peaux hypersensibles, communément qualifiées par le terme d'intolérantes, et/ou sont susceptibles de provoquer par leur emploi répété des réactions allergiques - on parle alors de peaux allergiques. Il s'avère ainsi souhaitable de les éliminer de la constitution des compositions à usage topique.

En particulier, la peau est recouverte d'un film protecteur, appelé film hydrolipidique de surface. Ce film en constitue la barrière la plus externe, ainsi que la plus fragile, la plus perturbée et la plus représentative de la santé de la peau. Il est constitué en grande partie des corps gras excrétés par les glandes sébacées et des lipides provenant de la dégradation des cellules lors de la phase de kératinisation des cellules cornées, ainsi que de composés hydrophiles, tels que l'eau de la sueur, le glycérol, l'urée, les facteurs naturels d'hydratation de la peau, les sels, les métabolites de la flore cutanée, etc. Ce film de surface est très exposé et très sensible aux stress environnementaux, aux habitudes d'hygiène, et à l'état de la peau. Il s'avère important de préserver, et même d'améliorer, cette fonction barrière, et ce d'autant plus pour les peaux les plus sensibles. Et, s'il est connu que les populations à peaux intolérantes dont la barrière cutanée est fragilisée nécessitent des soins avec des agents hydrolipidiques physio-mimétiques, notamment des agents émollients et hydratants physiologiques, il est en outre important d'éviter la mise en contact avec la peau de toute substance susceptible de dégrader le film hydrolipidique de surface, telle qu'un agent tensioactif ou un conservateur.

Le document FR 2 963 250 décrit une émulsion de type huile dans l'eau comprenant un polymère réticulé d'acrylate/acrylate d'alkyle et au moins un polyacrylate, qui est exempte de tensioactif et de conservateur.

Il a été proposé par la déposante, dans le document de brevet WO-A-2013/007755, un procédé pour la stérilisation par infusion à ultra-haute température (UHT) des émulsions à usage cosmétique, dermatologique et/ou pharmaceutique. Ce procédé de stérilisation permet la préservation de telles émulsions pendant une longue durée, en conditionnement multidoses, sans avoir recours à des conservateurs. Il assure en outre le maintien de l'intégrité de ces émulsions, qui conservent, à l'issue de sa mise en œuvre, une bonne homogénéité de dispersion de la phase interne dans la phase externe.

Cependant, un tel procédé de stérilisation, qui comprend une étape de contact de l'émulsion avec de la vapeur d'eau à très haute température, et qui peut en outre comprendre une étape de traitement mécanique par cisaillement de l'émulsion, est susceptible de modifier les propriétés rhéologiques de l'émulsion, notamment d'entraîner une modification de sa viscosité, ainsi que, plus généralement, de modifier ses caractères organoleptiques, tels que son aspect ou sa texture.

Il a maintenant été découvert par les présents inventeurs qu'il est possible de former une composition topique du type comportant une phase interne discontinue dispersée de manière homogène dans une phase externe continue, qui non seulement est avantageusement exempte d'agent tensioactif, mais qui peut en outre être soumise à un tel procédé de stérilisation par infusion à ultra-haute température, si bien qu'elle peut être également exempte de tout conservateur, sans dégradation de ses propriétés, et même avec une amélioration de certaines d'entre elles. Ceci est avantageusement permis par un choix particulier des constituants de cette composition, plus particulièrement par la combinaison d'un agent stabilisant de la dispersion de la phase interne dans la phase externe et d'un facteur de consistance d'un type particulier. Ce facteur de consistance est choisi parmi les alcools gras, tels que l'alcool béhénique.

Après avoir été soumise à un tel procédé de stérilisation par infusion à ultra-haute température, la composition répondant à cette constitution particulière ne présente aucune perte de sa stabilité ni aucune dégradation de ses propriétés, et ce malgré le fait qu'elle ne contient aucun agent tensioactif. En outre, la viscosité de la composition a pu être ajustée, et la composition présente même des propriétés organoleptiques améliorées, en particulier une microstructure plus fine.

Plus précisément, la mise en œuvre, sur une composition présentant une telle constitution particulière, de la technique de stérilisation par infusion à ultra-haute température, a pour effet, de manière tout à fait surprenante, de réduire la taille des gouttelettes de phase interne dispersées dans la phase externe, ainsi que d'améliorer l'homogénéité de la dispersion de ces gouttelettes dans la phase externe. De telles caractéristiques confèrent à la composition une texture particulièrement agréable au contact de la peau, et moins collante, plus facile et plus rapide à étaler que les compositions classiques contenant des agents hydrolipidiques physio-mimétiques.

La présente invention vise ainsi à proposer une composition cosmétique, dermatologique et/ou pharmaceutique, en particulier destinée à une application par voie topique, du type comportant une phase interne discontinue dispersée de manière homogène dans une phase externe continue, qui ne contienne pas d'agents susceptibles d'irriter la peau ou de générer des réactions allergiques, et qui soit compatible avec une stérilisation par infusion à ultra-haute température, c'est-à-dire qui conserve ses caractéristiques après une telle stérilisation, notamment sa stabilité et sa viscosité, et voire même qui voit certaines de ses caractéristiques améliorées.

Un objectif supplémentaire de l'invention est que cette composition soit physio-compatible, et qu'elle se rapproche notamment au plus près de la composition originelle du film hydrolipidique de la peau, de sorte à renforcer la protection naturelle de surface conférée par ce film, en particulier pour les peaux hypersensibles, à faible degré de tolérance.

A cet effet, il est proposé selon la présente invention une composition cosmétique, dermatologique et/ou pharmaceutique, en particulier à usage topique, comportant une phase interne discontinue dispersée dans une phase externe continue, l'une de ces phases, de préférence la phase externe, étant une phase aqueuse et l'autre de ces phases, de préférence la phase interne, étant une phase huileuse. Cette composition est notamment destinée à être soumise à un procédé de stérilisation par infusion à ultra-haute température, ou a été soumise à un tel procédé.

La composition selon l'invention est telle que définie dans la revendication 1, et contient 0,3 à 0,7 % en poids, par rapport au poids total de la composition, d'un polymère réticulé d'acrylate d'alkyle en C10-C30 et d'acide acrylique ou méthacrylique en tant que polymère polyacrylique, ainsi que 2 à 7 % en poids, par rapport au poids total de la composition, d'un alcool gras en tant que facteur de consistance apte à augmenter la viscosité de la composition par un phénomène d'épaississement physique semi-solide, insoluble dans l'eau et liposuble, c'est-à-dire soluble dans les corps gras. La composition est en outre exempte d'agent tensioactif et de conservateur.

Le polymère polyacrylique (polymère réticulé d'acrylate d'alkyle en C10-C30 et d'acide acrylique ou méthacrylique) est avantageusement apte à stabiliser la composition, plus particulièrement la dispersion de la phase interne dans la phase externe. Il est du type des polymères gélifiants présentant des zones hydrophobes (tels que les polymères associatifs communément désignés par l'expression polymères HASE, pour l'anglais Hydrophobic Alkali Swellable Emulsion). De tels composés gélifiants ne constituent en particulier pas des facteurs de consistance au sens de la présente invention.

Par agent tensioactif, on entend de manière classique en elle-même toute molécule amphiphile capable d'agir sur la tension interfaciale d'un milieu dispersé. La composition selon l'invention est avantageusement exempte de tel agent tensioactif, susceptible de solubiliser le film-hydrolipidique de la peau.

La composition selon l'invention est également dépourvue de conservateurs, susceptibles d'être responsables d'intolérances cutanées, notamment de tout ammonium quaternaire, éthanol, phénols, amidines, dérivés d'isothiazolone, esters parahydroxybenzoïques (connus sous le nom de parabens), etc.

Par conservateur, on entend dans la présente description toute substance apte à empêcher le développement de micro-organismes dans la composition, avantageusement par son action antimicrobienne propre. Le terme conservateur, au sens de la présente invention, englobe aussi bien les conservateurs *stricto sensu,* notamment tels que réglementairement listés (par exemple dans le Règlement CE n° 1223/2009 du Parlement Européen et du Conseil du 30 novembre 2009 relatif aux produits cosmétiques, définition article 2.1.I et Annexe V), que les substances dites « conservateur-like », non listées par la réglementation, mais présentant tout de même une fonction de conservateur. De telles substances sont susceptibles d'être présentes dans les formules cosmétiques pour des fonctions autres (parfumer, tonifier, raffermir, etc.), mais présentent également des propriétés antimicrobiennes, et empêchent ainsi la croissance des micro-organismes. Sont ainsi par exemple exclus de la composition selon l'invention, pour leur effet de conservateur, les composés tels que le caprylyl glycol, le pentylèneglycol, l'éthylhexylglycérine, etc.

Ainsi, le terme conservateur, au sens de la présente invention, englobe avantageusement les conservateurs listés dans l'Annexe V du Règlement CE n° 1223/2009 du Parlement Européen et du Conseil du 30 novembre 2009 relatif aux produits cosmétiques, ainsi que le caprylyl glycol, le pentylèneglycol et l'éthylhexylglycérine.

La composition selon l'invention, exempte d'agent tensioactif et de conservateur, se présente sous forme d'une dispersion de la phase interne dans la phase externe qui, grâce à l'action du polymère polyacrylique, est homogène, c'est-à-dire que des gouttelettes de phase interne sont réparties de manière sensiblement uniforme dans la phase externe, et stable. En outre, l'association de ce polymère polyacrylique avec ce facteur de consistance permet avantageusement à la composition selon l'invention d'être compatible avec un procédé de stérilisation par infusion à ultra-haute température, c'est-à-dire de présenter, à l'issue d'un tel procédé de stérilisation, une bonne stabilité et des propriétés rhéologiques, et plus généralement organoleptiques, améliorées.

On entend, par stabilité, une bonne homogénéité de dispersion de la phase interne, sous forme de microgouttelettes, dans la phase externe. Une composition stable est notamment une composition qui ne subit aucun phénomène de relargage, déphasage, précipitation, coalescence, floculation, crémage, etc. La composition selon l'invention est avantageusement telle que l'équilibre des phases qui la composent est maintenu, c'est-à-dire que la dispersion de la phase interne dans la phase externe reste homogène, à l'issue du procédé de stérilisation par infusion UHT, et ce malgré le fait que le polymère polyacrylique dont la présence est préconisée par la présente invention soit sensible au cisaillement. Il ne se produit notamment pas, après la mise en œuvre du procédé de stérilisation, y compris longtemps après, dans la composition selon l'invention, de phénomènes de relargage, déphasage, précipitation, coalescence, floculation, crémage, etc.

A l'issue d'un tel procédé de stérilisation, la composition selon l'invention peut en outre avantageusement être conservée pendant une longue période, malgré l'absence de conservateur.

Selon l'invention, le polymère polyacrylique est un copolymère réticulé d'acrylate d'alkyle en C10-C30 et d'acide acrylique ou méthacrylique, tel que les copolymères commercialisés sous les noms de Pemulen® TR-1 et TR-2, ou un mélange de tels copolymères.

Un tel copolymère, hautement réticulé, présente l'avantage, par rapport aux agents tensioactifs classiquement mis en œuvre dans les compositions cosmétiques, dermatologiques et/ou pharmaceutiques sous forme d'émulsion, non seulement de stabiliser la dispersion de la phase interne dans la phase externe, mais également de gélifier la composition. Il agit par encombrement stérique. Il possède de longues chaines hydrophiles qui forment un réseau de micro-gels autour des gouttelettes d'huile. Grâce à ses groupements carbonés de longue chaine (C10-C30), ce réseau tridimensionnel gélifié se maintient en place par répulsion physique, ce qui a pour effet de stabiliser les gouttelettes d'huile, et permet d'obtenir une composition topique homogène stable dans le temps, qui présente des caractéristiques proches de celles d'une émulsion sans toutefois mettre en œuvre d'agents tensioactifs.

En outre, contrairement aux agents tensioactifs, un tel copolymère ne pénètre pas dans la peau, ce qui réduit considérablement le risque qu'il génère une irritation au contact avec la peau.

Un tel copolymère est présent dans la composition dans une concentration comprise entre 0,3 et 0,7 %, par exemple entre 0,3 et 0,5 %, en poids par rapport au poids total de la composition.

Un facteur de consistance apte à augmenter la viscosité d'une composition par un phénomène d'épaississement physique semi-solide est défini dans la présente description de manière classique en elle-même, comme une substance solide à température ambiante et de point de fusion supérieur à 50 °C. Incorporé dans une phase huileuse à chaud, il retrouve à froid sa consistance semi-solide et confère à la phase huileuse une viscosité et une consistance qui sont réglées par son pourcentage dans la composition.

Plus particulièrement, le facteur de consistance selon l'invention est une substance du type non hydrosoluble, et liposoluble.

Sont en particulier exclus de la définition d'un facteur de consistance selon la présente invention, les agents gélifiants, notamment hydrosolubles et/ou non liposolubles, tels que la gomme xanthane, le polyacrylate de sodium, les gélifiants tels que les polymères HASE, etc.

Dans la composition selon l'invention, en association avec le polymère polyacrylique, ce facteur de consistance permet, à l'issue du procédé de stérilisation, d'obtenir la viscosité de la composition souhaitée. Il ne sera pas préjugé ici du mécanisme sous-tendant l'obtention d'un tel effet avantageux. On peut cependant supposer que l'épaississement physique de la composition contenant le polymère polyacrylique par le facteur de consistance soluble dans la phase huileuse, mais non dans la phase aqueuse, participe à empêcher que le stress mécanique, induit sur la composition par l'étape de cisaillement du procédé de stérilisation, ne casse le réseau gélifié formé avec les gouttelettes de phase interne par le polymère polyacrylique, et ne modifie ainsi la rhéologie de la composition. Les phases aqueuse et huileuse de la composition sont en outre maintenues intimement liées durant de longues périodes, compatibles avec la durée de vie d'une composition cosmétique, dermatologique et/ou pharmaceutique, et ce quelles que soient les proportions respectives de chacune de ces phases dans la composition.

En outre, les propriétés organoleptiques de la composition se trouvent avantageusement améliorées à l'issue du procédé de stérilisation. En particulier, comme exposé ci-avant, les microgouttelettes de phase interne dispersées dans la phase externe y sont plus fines, et dispersées de manière plus homogène, que dans la composition avant stérilisation.

Le facteur de consistance est choisi parmi les alcools gras ; l'alcool béhénique, également nommé docosanol, présentant une chaine carbonée à 22 atomes de carbone, est particulièrement préféré dans le cadre de l'invention. En particulier, l'alcool béhénique s'avère d'un intérêt particulier en ce qui concerne les propriétés de physio-compatibilité de la composition selon l'invention. Il s'agit en effet d'un alcool gras dérivé des chaines d'acides gras de taille importante les plus fréquemment retrouvés dans les lipides intercornéocytaires entrant dans la constitution des couches supérieures de l'épiderme (*Stratum corneum*).

Le facteur de consistance est présent dans la composition dans une concentration comprise entre 2 et 7 %, en poids par rapport au poids total de la composition.

Les concentrations relatives du polymère polyacrylique et du facteur de consistance dans la composition sont en outre choisies de manière à obtenir, à l'issue du traitement de stérilisation, la viscosité souhaitée pour la composition, le facteur de consistance permettant avantageusement de minimiser les conséquences, sur la viscosité de la composition, de l'effet du traitement de stérilisation par infusion à ultra-haute température sur les propriétés gélifiantes du polymère polyacrylique.

La composition selon l'invention, exempte d'agent tensioactif et de conservateur, peut se présenter sous forme d'une dispersion homogène d'une phase huileuse dans une phase aqueuse, du type huile-dans-eau. Ainsi, elle peut être telle que la phase interne est une phase huileuse et la phase externe est une phase aqueuse.

Autrement, elle peut se présenter sous forme d'une dispersion homogène d'une phase aqueuse dans une phase huileuse, du type eau-dans-huile.

La composition selon l'invention peut en outre contenir un agent actif par voie topique, ou un mélange de tels agents actifs.

Cet agent actif peut notamment consister en un agent hydratant, de préférence physiologique, notamment contenu dans la phase aqueuse.

Cet agent hydratant, également désigné sous les termes d'humectant physiologique, permet avantageusement de préserver le film hydrolipidique de surface et de maintenir une bonne hydratation de surface de la peau.

A titre de tel agent hydratant physiologique pouvant entrer dans la constitution de la composition selon l'invention, on peut citer le glycérol (ou glycérine), qui présente notamment les avantages, d'une part, d'être formé naturellement lors de l'hydrolyse naturelle des triglycérides du film hydrolipidique de surface et des lipides intercornéocytaires du *Stratum corneum,* et d'autre part, de présenter une forte capacité de rétention en eau à la surface cutanée, et donc une forte capacité de maintien d'une hydratation de surface.

Au titre d'agent actif par voie topique, la composition selon l'invention peut également ou autrement comporter un agent émollient, de préférence de structure proche de ceux retrouvés physiologiquement, tel que des triglycérides. Cet agent émollient peut en particulier être contenu dans la phase huileuse.

Les proportions de chacun de cet agent hydratant et de cet agent émollient dans la composition sont classiques en elles-mêmes, et peuvent par exemple être comprises entre 5 et 10 % en poids, par rapport au poids total de la composition, pour l'agent hydratant, notamment le glycérol, et entre 5 et 12% en poids, par rapport au poids total de la composition, pour l'agent émollient, notamment les triglycérides d'acides caprylique / caprique.

La phase aqueuse et la phase huileuse sont quant à elles mises en œuvre dans des proportions relatives habituelles dans le domaine cosmétique, dermatologique ou pharmaceutique, en fonction de la forme galénique souhaitée pour la composition. La détermination de ces proportions relatives entre dans les compétences de l'homme du métier.

Selon une caractéristique particulièrement avantageuse de l'invention, la composition comporte de préférence un agent hydratant physiologique, contenu dans la phase aqueuse, et un agent émollient physiologique, contenu dans la phase huileuse.

La composition selon l'invention, répondant à une telle caractéristique, participe avantageusement, lorsqu'elle est appliquée sur la surface cutanée, à un rééquilibrage en surface des lipides et des humectants naturels du film hydrolipidique de surface.

En outre, un peu plus profondément, dans les couches supérieures de l'épiderme, elle permet de combler les espaces intercornéocytaires avec des lipides adaptés sans provoquer d'occlusion. Ceci est notamment dû à la grande finesse des microgouttelettes de phase huileuse composant l'émulsion, en particulier à l'issue du procédé de stérilisation par infusion à ultra-haute température, qui permet une pénétration plus facile de ces microgouttelettes entre les cornéocytes, et un comblement plus rapide et efficace des lamelles lipidiques protectrices de la peau. Le comblement lipidique cornéocytaire en est d'autant plus rémanent.

La composition selon l'invention contient de préférence moins de 10 ingrédients au total.

Avantageusement, aucun de ces ingrédients n'est du type à risque d'intolérance, ou du type modifiant la biologie de la peau. Tous sont de préférence des ingrédients physio-compatibles, si bien qu'on peut alors qualifier la composition selon l'invention de physio-composition.

En particulier, la composition selon l'invention est préférentiellement dépourvue de parfum, de colorant, ou encore d'agent antimicrobien, bactéricide ou bactériostatique, fongistatique ou fongicide, si bien qu'elle respecte l'équilibre cutané. Elle est de préférence également dépourvue, parmi les adjuvants habituels pour les compositions cosmétiques, dermatologiques et/ou pharmaceutiques, de glycols (autre que le glycérol), de silicones, d'huiles minérales, de lanoline et ses dérivés, d'antioxydants de type BHT (hydroxytoluène butylé) ou BHA (hydroxyanisol butylé), de chélatants tels que l'EDTA (éthylène diamine tétra acétique), etc.

Le choix des ingrédients entrant dans la constitution de la phase huileuse est en outre préférentiellement limité aux ingrédients qui, outre le fait qu'ils soient cosmétiquement, dermatologiquement et/ou pharmaceutiquement compatibles, présentent en outre une similarité avec les lipides du film hydrolipidique de surface et du *Stratum Corneum,* et ne sont pas occlusifs.

Ainsi, la phase huileuse contient de préférence un ou plusieurs ingrédients choisis parmi les matières grasses d'origine végétale, pour leur teneur en acides gras et triglycérides physiologiques, telles que le beurre de karité, riche en acides gras oméga-6 et oméga-9 et l'huile de carthame, riche en acides gras oméga-6, le squalane, pour sa composition mimétique du sébum du film hydrolipidique de surface, les triglycérides de chaîne moyenne pour leur présence à l'état naturel dans le film hydrolipidique de surface et leur faible potentiel d'oxydation, etc.

La composition selon l'invention peut notamment se présenter sous une forme applicable par voie topique.

Par exemple, elle peut se présenter sous la forme galénique d'un lait, d'une crème, d'un masque ou d'un fluide.

La composition selon l'invention, sous forme de crème, peut par exemple répondre à la constitution suivante, la quantité de chacun des ingrédients étant exprimée en pourcentage en poids, par rapport au poids total de la composition :

| Phase aqueuse | |
|---|---|
| Eau déminéralisée ou thermale | 64 à 74 % |
| Glycérol | 5 à 10 % |

| Phase huileuse | |
|---|---|
| Huile de carthame | 5 à 10 % |
| Beurre de karité hydrogéné | 0 à 10 % |
| Triglycérides d'acides caprylique/caprique | 5 à 12 % |
| Alcool béhénique | 2 à 7 % |
| Polymère d'acrylate C10-C30 | 0,3 à 0,7 % |

Préférentiellement, la composition selon l'invention est stérile. On définit ici la stérilité de la composition de manière classique en elle-même, conformément à la norme NF EN 556 et à la pharmacopée européenne en vigueur, comme la probabilité d'un micro-organisme de proliférer dans la composition. Typiquement, cette probabilité, pour un produit stérile, est inférieure à $10^{-6}$.

La composition selon l'invention présente de préférence un niveau de stérilité tel qu'obtenu pour une valeur stérilisatrice F0 = 22 minutes. Un tel niveau de stérilité peut notamment être obtenu en soumettant la composition selon l'invention à un traitement de stérilisation par infusion à ultra-haute température tel que décrit dans le document WO-A-2013/007755.

La valeur stérilisatrice du procédé de stérilisation permettant d'atteindre le niveau de stérilité souhaité pour la composition, F0, dont la méthode de détermination est définie par la pharmacopée européenne en vigueur, correspond à un temps, exprimé en minutes, quantifiant l'effet létal de la chaleur humide à 121 °C sur les micro-organismes viables. L'effet létal est mesuré par rapport à un germe de référence, le *Geobacillus stearothermophilus* sporulé. Ce germe est particulièrement résistant et tolérant à la chaleur. La valeur de F0 est donnée par la formule :

$$F0 = t.10^{(T-121/z)}$$

où t est le temps de traitement exprimé en minutes,

z a la dimension d'une température et est défini par la résistance thermique du microorganisme considéré. La valeur de z est définie expérimentalement en regard d'un paramètre D. D est un temps de réduction décimal et mesure le temps, à une température donnée, ici à 121 °C, pour réduire la concentration du microorganisme considéré de 90 %. Pour *Geobacillus stearothermophilus,* D est égal à 1 min. Ainsi, z est la variation de température qui modifie la valeur de D d'un facteur 10. Pour *Geobacillus stearothermophilus,* z est égal à 10 °C. Ces facteurs D et z sont fonction du milieu et varient notamment selon le type de composition,

T est la température du traitement.

Ainsi, un traitement de valeur stérilisatrice F0 égale à 22 minutes, est un traitement de 22 minutes à 121 °C (394 K), ou encore un traitement de 36 secondes à 135 °C (408 K).

La composition selon l'invention est ainsi susceptible d'être obtenue par un procédé comprenant, après une étape de dispersion de la phase interne dans la phase externe, par mélange de la phase aqueuse et de la phase huileuse, contenant chacune les ingrédients adéquats, une étape de stérilisation par infusion à ultra-haute température, comme décrit ci-après.

Selon un autre objet, la présente invention concerne ainsi également un procédé de préparation d'une composition selon l'invention, répondant à l'une ou plusieurs des caractéristiques décrites ci-avant. Ce procédé comprend des étapes de :

- préparation de la phase aqueuse et de la phase huileuse, la phase huileuse contenant l'alcool gras en tant que facteur de consistance et au moins une de la phase aqueuse et de la phase huileuse, par exemple la phase huileuse, contenant le polymère réticulé d'acrylate d'alkyle en C10-C30 et d'acide acrylique ou méthacrylique en tant que polymère polyacrylique,
- mélange de la phase aqueuse et de la phase huileuse, de sorte à former une composition dans laquelle l'une des phases est dispersée de manière homogène dans l'autre phase,
- stérilisation de la composition ainsi formée par infusion à haute température.

Selon des modes de mise en œuvre particuliers de l'invention, l'étape de stérilisation de la composition par infusion à haute température comprend les étapes consistant à :

a. préchauffer progressivement la composition jusqu'à une température limite de stabilité de la composition, dite température de préchauffage, notamment de 55 °C ;
b. réaliser une stérilisation à ultra-haute température par infusion de la composition ainsi préchauffée comprenant :

   i. un chauffage à une température de stérilisation, notamment de 145 °C,

   ii. un maintien à la température de stérilisation, notamment pendant au moins 6 s,

   iii. un refroidissement sous vide à une température de fin de stérilisation, notamment de 50 °C,

c. refroidir progressivement sous agitation à une température de stockage, par exemple d'environ 30 °C, notamment par paliers, l'écart de température entre deux paliers étant de préférence inférieur ou égal à 15 °C.

Les étapes de préchauffage et le refroidissement sont préférentiellement mises en œuvre au moyen d'échangeurs thermiques à parois raclées, comme décrit dans le document de brevet WO-A-2013/007755.

Dans des modes de mise en œuvre particuliers de l'invention, l'étape de stérilisation comprend en outre une étape de traitement mécanique, dit de malaxage, par cisaillement de la composition après l'étape b de stérilisation à ultra-haute température.

Elle peut en outre comprendre une étape de malaxage de la composition après l'étape c de refroidissement progressif jusqu'à la température de stockage.

Un autre aspect de l'invention concerne l'utilisation non-thérapeutique d'une composition selon l'invention, répondant à l'une ou plusieurs des caractéristiques ci-avant, pour le traitement cosmétique de la peau, des muqueuses et/ou des cheveux.

A cet effet, la composition selon l'invention peut être appliquée sur la peau, les muqueuses et/ou les cheveux par voie topique.

Ainsi, un procédé de traitement cosmétique de la peau, des muqueuses et/ou des cheveux comprend d'application par voie topique, sur la peau, les muqueuses et/ou les cheveux, d'une composition selon l'invention.

La présente invention concerne par ailleurs l'utilisation d'une composition selon l'invention pour le traitement curatif et/ou préventif de maladies de la peau, des muqueuses et/ou des cheveux.

Les caractéristiques et avantages de l'invention apparaîtront plus clairement à la lumière des exemples de mise en œuvre ci-après, fournis à simple titre illustratif et nullement limitatifs de l'invention, avec l'appui des figures 1 à 2, dans lesquelles :

- la figure 1 montre des images obtenues au microscope, avec un grossissement de 40 fois, d'une composition conforme à la présente invention sous forme d'une crème, a) avant, et b) après, un traitement de stérilisation par infusion à haute température ;

- et la figure 2 montre des images obtenues au microscope, avec un grossissement de 40 fois, d'une composition conforme à la présente invention sous forme d'un lait, a) avant, et b) après, un traitement de stérilisation par infusion à haute température.

EXEMPLE 1

Une crème conforme à l'invention, particulièrement adaptée aux peaux hypersensibles et allergiques, est préparée par mélange des phases ci-dessous. Pour chaque composant, il est indiqué le pourcentage en poids, par rapport au poids total de la composition.

<u>Phase aqueuse</u>

| | |
|---|---|
| Eau déminéralisée ou thermale | qsp 100 % |
| Glycérol | 5-10% |

Le mélange est porté à 40 °C.

<u>Phase huileuse</u>

| | |
|---|---|
| Huile de carthame | 5 - 10 % |
| Beurre de karité hydrogéné | 5 - 10 % |
| Triglycérides d'acides caprylique/caprique | 5 - 12 % |
| Alcool béhénique | 2 - 7 % |

Le mélange est porté à 80 °C, puis il est ajouté l'ingrédient suivant :

Pemulen® TR-2  0,3 - 0,7 %

La phase huileuse est introduite progressivement dans la phase aqueuse, de sorte à la disperser de manière homogène dans cette dernière, puis le mélange est refroidi à 28 °C, et filtré.

On obtient à l'issue de ces étapes une composition dans laquelle de fines gouttelettes de phase huileuse sont dispersées de manière relativement homogène dans la phase aqueuse. La viscosité de cette composition est mesurée à 11800 cps.

La composition ainsi obtenue est soumise à un traitement de stérilisation par infusion à haute température, suivant le procédé décrit dans le document WO-A-2013/007755, au moyen d'un dispositif de stérilisation tel que décrit dans ce document, comportant un dispositif de stérilisation à ultra-haute température (UHT) par infusion, des échangeurs thermiques à parois raclées pour le préchauffage et le refroidissement de l'émulsion, et des moyens de traitement mécanique par cisaillement de la composition en sortie du dispositif de stérilisation UHT. Les paramètres opératoires sont les suivants :

a. préchauffage progressif de la composition jusqu'à une température de préchauffage de 55 °C ;

b. stérilisation par infusion de la composition ainsi préchauffée comprenant :

i. le chauffage à une température de stérilisation de 145 °C,

ii. le maintien à cette température de stérilisation pendant 6 s,

iii. le refroidissement sous vide à une température de fin de stérilisation de 50 °C,

c. refroidissement progressif sous agitation jusqu'à une température de stockage de 30 °C, via un palier à 40 °C.

La fréquence des échangeurs thermiques est de 50 Hz.

A l'issue de ce procédé de stérilisation, l'aspect de la composition est évalué. Cet aspect est d'un blanc très intense et brillant. Le ressenti sur la peau est celui d'une crème légère et fine, facile à étaler et pénétrant rapidement dans la peau. La viscosité est mesurée à 7054 cps.

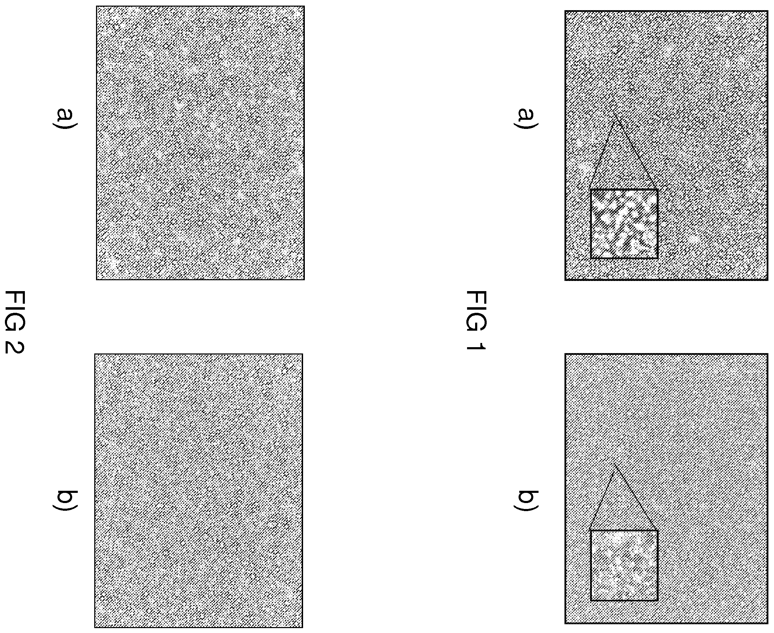

Il est réalisé une observation de la composition au microscope (microscope type Leica ICC50HD), avec un grossissement de 40 fois. L'image obtenue est montrée sur la <u>figure 1</u> (en b)). A titre de comparaison, il est également montré sur cette figure, en a), l'image obtenue par observation au microscope, au même grossissement, de la composition avant le traitement de stérilisation par infusion à haute température. On y observe clairement, notamment sur la vue partielle agrandie, que les gouttelettes de phase huileuse dispersées dans la phase aqueuse présentent une taille plus réduite après le traitement de stérilisation. Ces gouttelettes sont en outre dispersées de manière plus homogène dans la phase aqueuse.

La taille de ces gouttelettes a été évaluée au moyen d'une lame graduée installée sur le microscope, avant et

**EP 3 200 760 B1**

après le traitement de stérilisation. Les valeurs obtenues sont les suivantes : avant le traitement de stérilisation, la taille des gouttelettes est comprise entre 5 et 8 μm ; après le traitement de stérilisation, la taille des gouttelettes est comprise entre 2 et 3 μm. La taille des gouttelettes de phase huileuse dispersées dans la phase aqueuse est ainsi bien inférieure, et qui plus est avec une distribution de taille plus étroite, après stérilisation par infusion UHT, qu'avant une telle stérilisation.

Le procédé de stérilisation a ainsi non seulement permis de s'affranchir de l'adjonction de conservateurs dans la composition, mais il a également induit une amélioration des propriétés organoleptiques de cette composition.

EXEMPLE 2

Un lait démaquillant conforme à l'invention, particulièrement adapté aux peaux hypersensibles et allergiques, est préparé comme décrit dans l'Exemple 1 ci-dessus, à la différence que les phases aqueuses et huileuses présentant la composition suivante (pour chaque composant, il est indiqué le pourcentage en poids, par rapport au poids total de la composition) :

| Phase aqueuse | |
|---|---|
| Eau déminéralisée ou thermale | 77 à 79 % |
| Glycérol | 2 à 10 % |

| Phase huileuse | |
|---|---|
| Squalane | 5 à 10 % |
| Triglycérides d'acides caprylique/caprique | 5 à 12 % |
| Alcool béhénique | 2 à 7 % |
| Polymère d'acrylate en C10-C30 | 0,3 à 0,5 % |

A l'issue du procédé de stérilisation, on obtient une composition dans laquelle de très fines gouttelettes de phase huileuse sont dispersées de manière très homogène dans la phase aqueuse.

Il est réalisé une observation de la composition au microscope, avec un grossissement de 40 fois. L'image obtenue est montrée sur la figure 2 (en b)). A titre de comparaison, il est également montré sur cette figure, en a), l'image obtenue par observation au microscope, au même grossissement, de la composition avant le traitement de stérilisation par infusion à haute température. On y observe clairement que la taille des gouttelettes de phase huileuse dispersées dans la phase aqueuse est plus réduite après le traitement de stérilisation. Les gouttelettes y sont en outre dispersées de manière plus homogène.

EXEMPLE 3

Une crème conforme à l'invention, particulièrement adaptée aux peaux hypersensibles et allergiques, est préparée par mélange des phases ci-dessous. Pour chaque composant, il est indiqué le pourcentage en poids, par rapport au poids total de la composition.

| Phase aqueuse | |
|---|---|
| Eau déminéralisée ou thermale | qsp 100 % |
| Glycérol | 5 - 10 % |
| Pemulen® TR-2 | 0,3 - 0,7 % |

| Phase huileuse | |
|---|---|
| Huile de carthame | 5 - 10 % |
| Beurre de karité hydrogéné | 5 - 10 % |
| Triglycérides d'acides caprylique/caprique | 5 - 12 % |
| Alcool béhénique | 2 - 7 % |

La composition ainsi obtenue est soumise à un traitement de stérilisation par infusion à haute température, suivant le procédé décrit dans l'Exemple 1 ci-avant.

**Revendications**

1. Composition cosmétique, dermatologique et/ou pharmaceutique, comportant une phase interne discontinue dispersée dans une phase externe continue, l'une desdites phases étant une phase aqueuse et l'autre desdites phases étant une phase huileuse, ladite composition étant exempte d'agent tensioactif et de conservateur, **caractérisée en ce qu'**elle contient 0,3 à 0,7 % en poids, par rapport au poids total de la composition, d'un polymère réticulé d'acrylate d'alkyle en C10-C30 et d'acide acrylique ou méthacrylique, et 2 à 7 % en poids, par rapport au poids total de la composition, d'un alcool gras.

2. Composition selon la revendication 1, dans laquelle l'alcool gras est l'alcool béhénique.

3. Composition selon l'une quelconque des revendications 1 à 2, dans laquelle la phase interne est une phase huileuse et la phase externe est une phase aqueuse.

4. Composition selon l'une quelconque des revendications 1 à 3, contenant au moins un agent actif par voie topique.

5. Composition selon la revendication 4, contenant un agent hydratant.

6. Composition selon l'une quelconque des revendications 4 à 5, contenant un agent émollient.

7. Composition selon l'une quelconque des revendications 1 à 6, **caractérisée en ce qu'**elle est stérile selon la norme EN 556 et la pharmacopée européenne.

8. Composition selon l'une quelconque des revendications 1 à 7, contenant moins de 10 ingrédients au total.

9. Composition selon l'une quelconque des revendications 1 à 8, se présentant sous une forme applicable par voie topique.

10. Composition selon l'une quelconque des revendications 1 à 9, sous forme d'un lait, d'une crème, d'un masque ou d'un fluide.

11. Procédé de préparation d'une composition selon l'une quelconque des revendications 1 à 10, **caractérisé en ce qu'**il comprend des étapes de :

    - préparation de la phase aqueuse et de la phase huileuse, ladite phase huileuse contenant ledit alcool gras et au moins une de ladite phase aqueuse et de ladite phase huileuse contenant ledit polymère réticulé d'acrylate d'alkyle en C10-C30 et d'acide acrylique ou méthacrylique,
    - mélange de ladite phase aqueuse et de ladite phase huileuse, de sorte à former une composition dans laquelle l'une desdites phases est dispersée de manière homogène dans l'autre desdites phases et,
    - une étape de stérilisation de la composition formée par infusion à haute température.

12. Procédé de préparation selon la revendication 11, selon lequel l'étape de stérilisation de la composition comprend les étapes consistant à :

    a. préchauffer progressivement la composition jusqu'à une température limite de stabilité de l'émulsion ;
    b. réaliser une stérilisation à ultra-haute température par infusion de la composition ainsi préchauffée comprenant :

       i. un chauffage à une température de stérilisation,
       ii. un maintien à la température de stérilisation,
       iii. un refroidissement sous vide à une température de fin de stérilisation,

    c. refroidir progressivement sous agitation à une température de stockage.

13. Utilisation non-thérapeutique d'une composition selon l'une quelconque des revendications 1 à 10 pour le traitement cosmétique de la peau, des muqueuses et/ou des cheveux.

14. Utilisation selon la revendication 13, selon laquelle ladite composition est appliquée sur la peau, les muqueuses et/ou les cheveux par voie topique.

15. Composition selon l'une quelconque des revendications 4 à 10, pour son utilisation pour le traitement curatif et/ou préventif de maladies de la peau, des muqueuses et/ou des cheveux.

**Patentansprüche**

1. Kosmetische, dermatologische und/oder pharmazeutische Zusammensetzung, die eine diskontinuierliche innere Phase beinhaltet, die in einer kontinuierlichen äußeren Phase dispergiert ist, wobei eine der Phasen eine wässrige Phase ist und die andere der Phasen eine ölige Phase ist, wobei die Zusammensetzung frei von Tensid und Konservierungsmittel ist,
   **dadurch gekennzeichnet, dass** sie 0,3 bis 0,7 Gew.-% in Bezug auf das Gesamtgewicht der Zusammensetzung eines vernetzten Polymers aus C10-C30 Alkyl Acrylat und Acryl- oder Methacrylsäure, und 2 bis 7 Gew.-% in Bezug auf das Gesamtgewicht der Zusammensetzung eines Fettalkohols enthält.

2. Zusammensetzung nach Anspruch 1, wobei der Fettalkohol Behenalkohol ist.

3. Zusammensetzung nach einem der Ansprüche 1 bis 2, wobei die innere Phase eine ölige Phase ist und die äußere Phase eine wässrige Phase ist.

4. Zusammensetzung nach einem der Ansprüche 1 bis 3, die mindestens einen Wirkstoff auf topischem Weg enthält.

5. Zusammensetzung nach Anspruch 4, die ein Befeuchtungsmittel enthält.

6. Zusammensetzung nach einem der Ansprüche 4 bis 5, die ein Aufweichungsmittel enthält.

7. Zusammensetzung nach einem der Ansprüche 1 bis 6, **dadurch gekennzeichnet, dass** sie gemäß der Norm EN 556 und dem Europäischen Arzneibuch steril ist.

8. Zusammensetzung nach einem der Ansprüche 1 bis 7, die insgesamt weniger als 10 Zutaten enthält.

9. Zusammensetzung nach einem der Ansprüche 1 bis 8, die sich in einer auf topischem Weg anwendbaren Form darstellt.

10. Zusammensetzung nach einem der Ansprüche 1 bis 9, in Form einer Milch, einer Creme, einer Maske oder eines Fluids.

11. Verfahren zur Herstellung einer Zusammensetzung nach einem der Ansprüche 1 bis 10, **dadurch gekennzeichnet, dass** es die folgenden Schritte umfasst:

    - Herstellung der wässrigen Phase und der öligen Phase, wobei die ölige Phase den Fettalkohol enthält und mindestens eine der wässrigen Phase und der öligen Phase das vernetzte Polymer aus C10-C30 Alkyl Acrylat und Acryl- oder Methacrylsäure enthält,
    - Mischen der wässrigen Phase und der öligen Phase, um eine Zusammensetzung zu bilden, in der eine der Phasen in homogener Form in der anderen der Phasen dispergiert ist, und
    - einen Schritt zum Sterilisieren der Zusammensetzung, der durch Infusion bei hoher Temperatur gebildet wird.

12. Verfahren zur Herstellung nach Anspruch 11, wobei der Schritt zum Sterilisieren der Zusammensetzung die Schritte umfasst, die darin bestehen:

    a. die Zusammensetzung progressiv bis auf eine Stabilitätsgrenztemperatur der Emulsion vorzuwärmen;
    b. eine Sterilisation bei extrem hoher Temperatur durch Infusion der so vorgewärmten Zusammensetzung zu realisieren, umfassend:

       i. Erwärmen auf eine Sterilisationstemperatur,
       ii. Halten auf der Sterilisationstemperatur,
       iii. Abkühlen unter Vakuum auf eine Sterilisationsendtemperatur,

c. progressiv unter Rühren auf eine Lagertemperatur abzukühlen.

13. Nicht therapeutische Verwendung einer Zusammensetzung nach einem der Ansprüche 1 bis 10 für die kosmetische Behandlung der Haut, der Schleimhäute und/oder der Haare.

14. Verwendung nach Anspruch 13, wobei die Zusammensetzung auf topischem Weg auf die Haut, die Schleimhäute und/oder die Haare angewendet wird.

15. Zusammensetzung nach einem der Ansprüche 4 bis 10, für deren Verwendung bei der kurativen und/oder vorbeugenden Behandlung von Krankheiten der Haut, der Schleimhäute und/oder der Haare.

**Claims**

1. Cosmetic, dermatological and/or pharmaceutical composition, comprising a discontinuous internal phase dispersed in a continuous external phase, one of said phases being an aqueous phase and the other of said phases being an oily phase, said composition being free of surfactant and of preservative,
   **characterized in that** it contains 0.3 to 0.7 % by weight, with respect to the total weight of the composition, of a crosslinked copolymer of $C_{10}$-$C_{30}$ alkyl acrylate and of acrylic or methacrylic acid, and 2 to 7 % by weight, with respect to the total weight of the composition, of a fatty alcohol.

2. Composition according to Claim 1, in which the fatty alcohol is behenyl alcohol.

3. Composition according to any one of Claims 1 to 2, in which the internal phase is an oily phase and the external phase is an aqueous phase.

4. Composition according to any one of Claims 1 to 3, containing at least one topically active agent.

5. Composition according to Claim 4, containing a moisturizing agent.

6. Composition according to any one of Claims 4 to 5, containing an emollient agent.

7. Composition according to any one of Claims 1 to 6, **characterized in that** it is sterile according to Standard EN 556 and the European pharmacopoeia.

8. Composition according to any one of Claims 1 to 7, containing fewer than 10 ingredients in total.

9. Composition according to any one of Claims 1 to 8, which is in a topically applicable form.

10. Composition according to any one of Claims 1 to 9, in the form of a milk, a cream, a mask or a fluid.

11. Method for preparing a composition according to any one of Claims 1 to 10, **characterized in that** comprises steps of:

    - preparing the aqueous phase and the oily phase, said oily phase containing said fatty alcohol, and at least one of said aqueous phase and said oily phase containing said crosslinked copolymer of $C_{10}$-$C_{30}$ alkyl acrylate and of acrylic or methacrylic acid,
    - mixing said aqueous phase and said oily phase, so as to form a composition in which one of said phases is homogeneously dispersed in the other of said phases and,
    - a step of sterilizing the composition formed, by high-temperature infusion.

12. Preparation method according to Claim 11, according to which the step of sterilizing the composition comprises the steps consisting in:

    a. gradually preheating the composition to an emulsion stability limit temperature;
    b. carrying out an ultrahigh-temperature infusion sterilization of the composition thus preheated, comprising:

        i. heating to a sterilization temperature,
        ii. maintaining at the sterilization temperature,

iii. cooling under vacuum to a sterilization end temperature,

c. gradually cooling with stirring to a storage temperature.

13. Non-therapeutic use of a composition according to any one of Claims 1 to 10 for the cosmetic treatment of the skin, of the mucous membranes and/or of the hair.

14. Use according to Claim 13, according to which said composition is topically applied to the skin, the mucous membranes and/or the hair.

15. Composition according to any one of Claims 4 to 10, for its use for the curative and/or preventive treatment of diseases of the skin, of the mucous membranes and/or of the hair.

a)                                                      b)

FIG 1

a)                                                      b)

FIG 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2963250

- WO 2013007755 A